(19) Europäisches Patentamt
European Patent Office
Office européen des brevets

(11) **EP 2 026 058 A1**

(12) **EUROPEAN PATENT APPLICATION**
published in accordance with Art. 153(4) EPC

(43) Date of publication:
**18.02.2009 Bulletin 2009/08**

(51) Int Cl.:
**G01N 21/35** (2006.01)  **A61B 5/1455** (2006.01)

(21) Application number: **06757177.8**

(22) Date of filing: **08.06.2006**

(86) International application number:
**PCT/JP2006/311530**

(87) International publication number:
**WO 2007/141859 (13.12.2007 Gazette 2007/50)**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(71) Applicant: **Omron Healthcare Co., Ltd.
Kyoto 615-0084 (JP)**

(72) Inventor: **TOKITA, Muneo
Kyoto-shi, Kyoto 615-0084 (JP)**

(74) Representative: **Wilhelms · Kilian & Partner
Patentanwälte
Eduard-Schmid-Strasse 2
81541 München (DE)**

(54) **BIOLOGICAL COMPONENT MEASUREMENT DEVICE CAPABLE OF ACCURATELY NONINVASIVELY MEASURING BIOLOGICAL COMPONENT**

(57)    A living body component measuring apparatus emits light of a first wavelength being specific to a measuring target component from a light emitting unit (11) onto a measured body (30), receives through a filter (15A, 15B) at a light receiving unit (17) the light of the first wavelength being the reflected light from the measured body (30) and light of a second wavelength that is blackbody radiation from the measured body (30) and that is not absorbed in the component, and calculates the concentration of the component using the amounts of the light.

FIG.5

## Description

TECHNICAL FIELD

The present invention relates to a living body component measuring apparatus and a living body component measuring sensor, and particularly, to a living body component measuring apparatus and a living body component measuring sensor that receive light from a living body and thereby non-invasively measure a living body component.

BACKGROUND ART

Measurement of the concentration of a specific component contained in a living body tissue as represented by blood, body fluid and the like of a subject has been practiced. Representatives of the specific measuring target component may be glucose, hemoglobin, oxyhemoglobin, trilaurin, cholesterol, albumin, uric acid and the like. In particular, blood sugar measurement has increased its importance as a self-management tool of diabetics. It is regarded that frequent measurement contributes to an improvement in QOL (Quality of Life) of a patient, and ultimately to prevention of heart disease, complication and the like.

As one living body component measuring apparatus, there is an apparatus employing an invasive method in which a living body tissue such as subcutaneous effusion or the like is sampled and the concentration of a specific component contained therein is measured (a semi-invasive type apparatus). This measurement method puts much burden on the subject. Accordingly, there has been proposed an apparatus of non-invasive type that uses optics, employing a non-invasive method in which reflected light from a living body or transmitted light transmitted through the living body is received, and the concentration of a specific component is calculated from the light characteristics. For example, when the glucose concentration in the blood is to be measured using such a measuring apparatus, analysis of spectrum of a near-infrared band or a mid-infrared band included in radiation light from a measured site (for example, an eardrum) provides the glucose concentration in the blood.

In the non-invasive type living body component measuring apparatus employing optics, the temperature of a subject is a very important parameter. With a conventional general non-invasive type living body component measuring apparatus, the temperature is measured by placing a temperature measuring element such as a thermocouple near the irradiated portion or at another place. However, there has been a problem that, contrary to the fact that the temperature at the site irradiated with the energy of the light must change, if only a little, the non-invasive type living body component measuring method is not capable of precisely capturing such change in the temperature.

Accordingly, Japanese Patent Laying-Open No. 10-258036 (hereinafter referred to as Patent Document 1) discloses a blood sugar meter, in which near-infrared light is emitted and the temperature is calculated from the change in the absorption of the light, so that the measurement of the temperature of the irradiated portion is achieved.

Japanese Patent National Publication No. 5-507866 (hereinafter referred to as Patent Document 2) discloses an apparatus that uses heat generated in irradiation for measuring a specific substance in the blood. This method is known as photoacoustic spectroscopy, in which an optical pulse is applied to a measurement target, upon which expansion and contraction due to heat occur at the measurement target, and a sound (change in the pressure) generated thereby is received by a pressure element. The apparatus of Patent Document 2 employing this method does not include a light receiving element, and it does not perform measurement in which an error in light absorption is corrected using heat as a parameter.

Patent Document 1: Japanese Patent Laying-Open No. 10-258036
Patent Document 2: Japanese Patent National Publication No. 5-507866

DISCLOSURE OF THE INVENTION

PROBLEMS TO BE SOLVED BY THE INVENTION

On the other hand, there has been a problem that the temperature calculated by the blood sugar meter of Patent Document 1 includes the effect of change in the absorption of light that is the measuring target, and therefore the temperature can hardly be calculated precisely and a precise measurement result may not always be obtained.

Also, there has been a problem that the apparatus of Patent Document 2 does not employ the measurement method in which an error in the light absorption is corrected using heat as a parameter, as described above, and therefore a measurement result may include the effect of heat and a precise measurement result may not always be obtained.

The present invention has been made in light of the foregoing problems, and an object thereof is to provide a living body component measuring apparatus and a living body component measuring sensor that precisely measure changes in the temperature of a measured body due to light emitted thereon, changes in the amount of the emitted light, and changes in the body temperature, thereby precisely measuring a living body component.

MEANS FOR SOLVING THE PROBLEMS

In order to achieve the aforementioned object, according to one aspect of the present invention, when a wavelength exhibiting specific absorption in a measuring target living body component is a first wavelength and a wavelength not exhibiting specific absorption in the living body component is a second wavelength, a living body component measuring apparatus includes: a first light emitting unit emitting light of the first wavelength onto a measured body; a light receiving unit receiving light radiated from the measured body when the light of the first wavelength is emitted from the first light emitting unit, and outputting a signal according to a received light amount; and a concentration calculating unit calculating concentration of the living body component based on the signal. The light receiving unit includes a first light receiving unit outputting a first signal according to a received light amount of the light of the first wavelength and a second light receiving unit outputting a second signal according to a received light amount of the light of the second wavelength. The concentration calculating unit calculates the concentration of the living body component based on the first signal and the second signal.

More specifically, preferably, the light of the first wavelength and the light of the second wavelength are mid-infrared light.

Preferably, the concentration calculating unit calculates the concentration of the living body component based on the first signal and the second signal before and after the light of the first wavelength is emitted.

Preferably, the second light receiving unit includes a filter transmitting the second wavelength, and receives the light of the second wavelength being transmitted through the filter from among light radiated from the measured body.

Preferably, the living body component measuring apparatus further includes: a second light emitting unit emitting the light of the second wavelength onto the measured body; and switching means for switching between emission by the first light emitting unit and emission by the second light emitting unit. The light receiving unit further includes a third light receiving unit outputting a third signal according to the received light amount of the light of the first wavelength radiated from the measured body and a fourth light receiving unit outputting a fourth signal according to the received light amount of the light of the second wavelength radiated from the measured body, when the light of the second wavelength is emitted from the second light emitting unit. The concentration calculating unit calculates the concentration of the living body component based on the first to fourth signals.

Alternatively, preferably, when a wavelength not exhibiting specific absorption in the living body component and being different from the second wavelength is a third wavelength, the living body component measuring apparatus further includes: a third light emitting unit emitting light of the third wavelength onto the measured body; and switching means for switching between emission by the first light emitting unit and emission by the third light emitting unit. The light receiving unit further includes a fifth light receiving unit outputting a fifth signal according to the received light amount of the light of the second wavelength radiated from the measured body and a sixth light receiving unit outputting a sixth signal according to a received light amount of the light of the third wavelength radiated from the measured body, when the light of the third wavelength is emitted from the third light emitting unit. The concentration calculating unit calculates the concentration of the living body component based on the first signal, the second signal, the fifth signal, and the sixth signal.

Alternatively, preferably, the living body component measuring apparatus further includes: a second light emitting unit emitting the light of the second wavelength onto the measured body; and switching means for switching between emission by the first light emitting unit and emission by the second light emitting unit at a time interval that is shorter than thermal response of the measured body. The light receiving body further includes a fourth light receiving unit outputting a fourth signal according to the received light amount of the light of the second wavelength radiated from the measured body when the light of the second wavelength is emitted from the second light emitting unit. The calculating unit calculates the concentration of the living body component based on a difference between the first signal and the fourth signal and a difference between the fourth signal and the second signal.

According to another aspect of the present invention, when a wavelength exhibiting specific absorption in a measuring target living body component is a first wavelength and a wavelength not exhibiting specific absorption in the living body component is a second wavelength, a living body component measuring sensor includes: a first light emitting unit emitting light of the first wavelength onto a measured body; and a light receiving unit receiving light radiated from the measured body when the light of the first wavelength is emitted from the first light emitting unit, and outputting a signal according to a received light amount. The light receiving unit includes a first light receiving unit outputting a first signal according to a received light amount of the light of the first wavelength and a second light receiving unit outputting a second signal according to a received light amount of the light of the second wavelength.

Preferably, the living body component measuring sensor further includes: a second light emitting unit emitting the light of the second wavelength onto the measured body; and switching means for switching between emission by the first light emitting unit and emission by the second light emitting unit. The light receiving unit further includes a third light receiving unit outputting a third signal according to the received light amount of the light of the first wavelength radiated from the measured body and a fourth light receiving unit outputting a fourth signal according to the received light amount of the light of the second wavelength radiated from the measured body, when the light of the second wavelength is emitted from the second light emitting unit.

Alternatively, preferably, when a wavelength not exhibiting specific absorption in the living body component and being different from the second wavelength is a third wavelength, the living body component measuring sensor further includes: a third light emitting unit emitting light of the third wavelength onto the measured body; and switching means for switching between emission by the first light emitting unit and emission by the third light emitting unit. The light receiving unit further includes a fifth light receiving unit outputting a fifth signal according to the received light amount of the light of the second wavelength radiated from the measured body and a sixth light receiving unit outputting a sixth signal according to a received light amount of the light of the third wavelength radiated from the measured body, when the light of the third wavelength is emitted from the third light emitting unit.

EFFECTS OF THE INVENTION

The living body component measuring apparatus and living body component measuring sensor according to the present invention detect, when measuring transmission and/or scattering reflection, transmission of light from a light source or reflected light having an absorption wavelength of a target component, and simultaneously, using natural radiation light radiated from the measured body at that timing, capture changes in the temperature of a portion when it is irradiated, to perform correction. The radiation light of a living body has its peak in the mid-infrared band. The relationship between its light amount and temperature is precise, as demonstrated by the commercialization of infrared ear or forehead thermometers.

As above, the living body component measuring apparatus and living body component measuring sensor according to the present invention measures natural radiation light during light emission thereon, thereby precisely measuring changes in the temperature of a measured body due to the light emitted thereon, variations in the amount of the emitted light, and changes in the body temperature. Thus, precise correction can be performed.

Additionally, the living body component measuring apparatus and living body component measuring sensor according to the present invention include two light sources, whereby changes in the measured body itself such as scattering coefficients, moisture, inhibiting substances that are not reflected in natural radiation light can also be corrected.

BRIEF DESCRIPTION OF THE DRAWINGS

Fig. 1 shows a specific example of a configuration of a living body component measuring apparatus according to an embodiment.
Fig. 2 shows an absorption spectrum of glucose as a specific example of a living body component.
Fig. 3 shows output at a light receiving unit when glucose scattering aqueous solution is irradiated with light having a wavelength of 9.6 um and radiation of light from the solution is detected through each of a 9.6 um filter and an 8.5 um filter.
Fig. 4 shows the relationship between glucose concentration and the output at light receiving unit 17.
Fig. 5 is an explanatory view of a configuration of a living body component measuring sensor 10 in a living body component measuring apparatus according to a first embodiment.
Fig. 6 is an explanatory view of a configuration of living body component measuring sensor 10 in a living body component measuring apparatus according to a variation of the first embodiment.
Fig. 7A is an explanatory view of a configuration of living body component measuring sensor 10 in a living body component measuring apparatus according to a second embodiment.
Fig. 7B is an explanatory view of a variation of living body component measuring sensor 10 in the living body component measuring apparatus according to the second embodiment.
Fig. 7C is an explanatory view of a variation of living body component measuring sensor 10 in the living body component measuring apparatus according to the second embodiment.
Fig. 8A is an explanatory view of a configuration of living body component measuring sensor 10 in a living body component measuring apparatus according to a variation of the second embodiment.
Fig. 8B is an explanatory view of living body component measuring sensor 10 in a living body component measuring apparatus according to a variation of the second embodiment.
Fig. 8C is an explanatory view of living body component measuring sensor 10 in a living body component measuring apparatus according to a variation of the second embodiment.
Fig. 9 is a first graph schematically showing changes in output V from light receiving unit 17.

Fig. 10 is a second graph schematically showing changes in output V from light receiving unit 17.

DESCRIPTION OF THE REFERENCE SIGNS

30 measured body, 10 living body component measuring sensor, 11, 11A, 11B light emitting unit, 12 mirror, 13 light guiding unit, 13A internal surface of the light guiding unit, 14A, 14B, 15, 15A, 15B, 15C, filter, 17 light receiving unit, 21 light receiving circuit, 23 control unit, 25 concentration outputting unit.

BEST MODES FOR CARRYING OUT THE INVENTION

In the following, referring to the drawings, embodiments of the present invention will be described. In the following description, identical parts and constituents are denoted by identical reference characters. Their labels and functions are also the same.

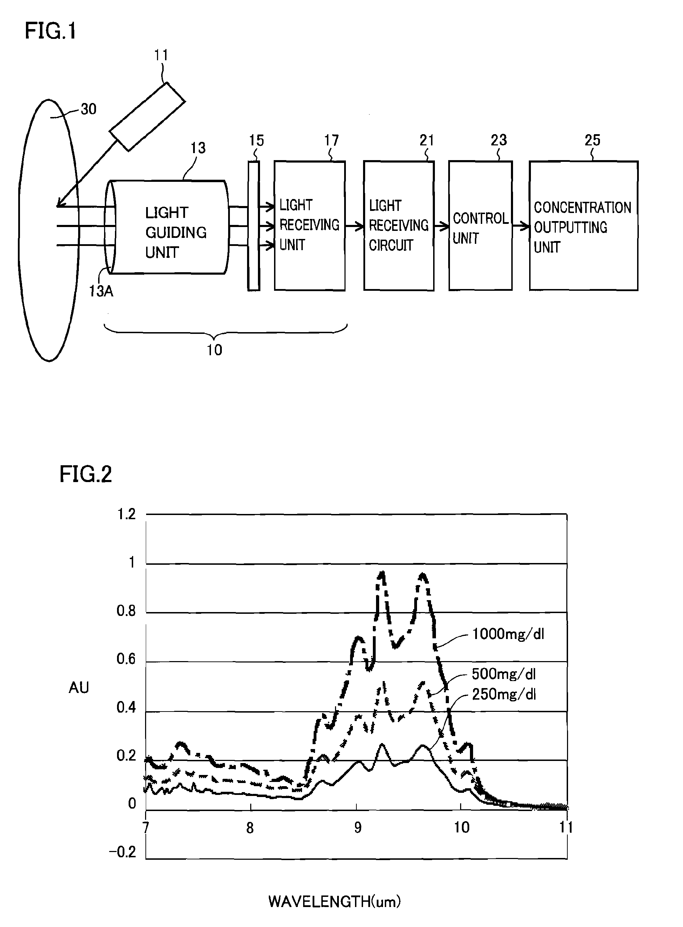

Referring to Fig. 1, a living body component measuring apparatus according to the present embodiment includes a living body component measuring sensor 10 receiving reflected light from a measured body 30, a control unit 23 receiving a sensing signal from living body component measuring sensor 10 via a light receiving circuit 21 and calculating the concentration of a prescribed living body component, and a concentration outputting unit 25 outputting the calculated concentration of the prescribed living body component.

Further, living body component measuring sensor 10 includes a light emitting unit 11 emitting light to measured body 30, a light guiding unit 13 guiding light radiated from measured body 30 to target direction, and a light receiving unit 17 including a light receiving element that receives the light guided by light guiding unit 13 via filter 15. It is noted that, in the present invention, a light receiving mechanism that includes filter 15 and light receiving unit 17 may be referred to as "a light receiving unit".

Light emitting unit 11 emits a mid-infrared light beam onto measured body 30. More preferably, it emits a light beam having a wavelength of 3 um - 11 um. A possible light source may be a heating element light source having a wide range of wavelengths (IR (infrared) light source using ceramic, alumina and the like) and various lasers of monochromatic light source (CO2 laser, YAG laser, quantum-cascade laser, and the like).

Suitable light guiding unit 13 is a pipe, fibers and the like, internal surface 13A of which forming a light guiding path is mirror-finished by gold plating, deposition of gold, aluminum and the like.

Possible light receiving elements included in light receiving unit 17 may be an MCT (MOS (Metal Oxide Semiconductor) Controlled Thyristor), a thermopile, a pyroelectric sensor, a bolometer and the like.

As shown in Fig. 2, irrespective of the concentration, a living body component specifically absorbs light of a specific wavelength. For glucose, it is known that the specific absorption wavelength is around 9.6 um. Since the absorption amount is different depending on the concentration, the concentration of the component can be obtained by measuring the light absorption amount.

With living body component measuring sensor 10, light having an absorption wavelength specific to the measuring target living body component is emitted onto measured body 30. When the measuring target living body component is glucose, emission of light having a wavelength of 9.6 um is preferable. The light emitted onto the measured body is scattered and absorbed therein, and part of the light is radiated as scattering reflected light from the same side as it has entered.

An object having a temperature emits light having a wavelength specific to its temperature and material. This emission of light is referred to as blackbody radiation. A living body of 37°C emits light having a wavelength with peak of about 9 um and in a range of 3 um - 50 um.

Accordingly, when light having an absorption wavelength specific to a living body component is emitted from light emitting unit 11 of living body component measuring sensor 10 onto measured body 30, the light received at light receiving unit 17, which has the same wavelength as the light emitted from light emitting unit 11, includes the scattering reflected light having been subjected to absorption by the living body component and the blackbody radiation corresponding to the temperature of measured body 30.

In the living body component measuring apparatus according to the present embodiment, when an absorption wavelength specific to the measuring target living body component is a first wavelength and a wavelength of light not absorbed by the living body component is a second wavelength, light of the first wavelength is emitted from light emitting unit 11 of living body component measuring sensor 10 onto measured body 30. Before and after such emission, light receiving unit 17 receives the light of the first wavelength and the light of the second wavelength radiated from measured body 30. Using changes in the light amount, control unit 23 calculates the concentration of the living body component. When the measuring target living body component is glucose, the first wavelength may be 9.6 um, and the second wavelength may be 8.5 um, 10.5 um or the like.

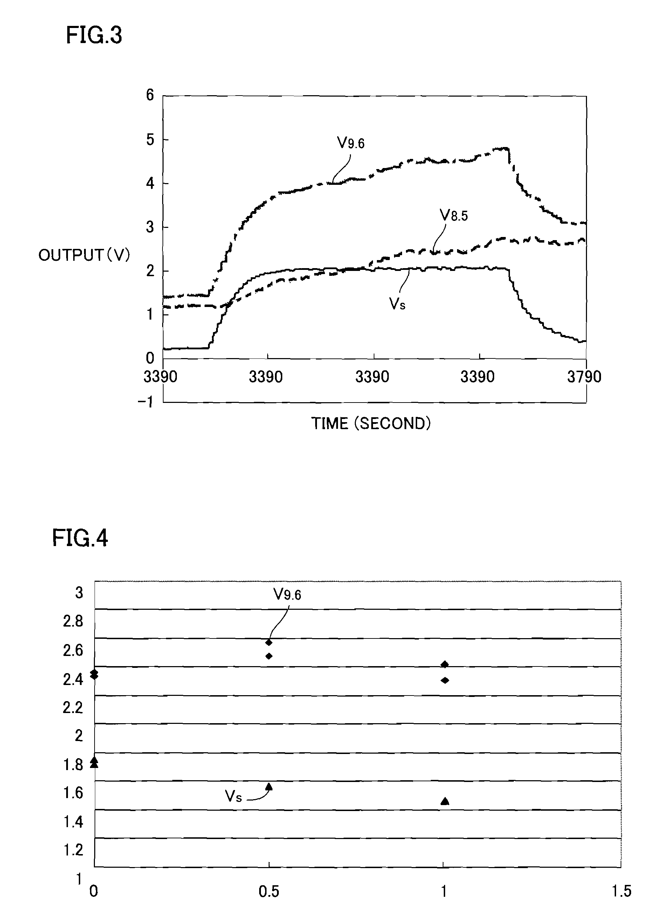

Referring to Fig. 3, light emitting unit 11 is a ceramic IR light source provided with a bandpass filter of 9.6 um. Measured body 30 is glucose scattering aqueous solution. Light having a wavelength of 9.6 um is emitted onto glucose scattering aqueous solution being measurement body 30. The light radiated from the solution is detected using each of filters 15 being 9.6 um filter and 8.5 filter. Fig. 3 shows the detected output $V_{9.6}$ from light receiving unit 17 with the wavelength of 9.6 um and output $V_{8.5(9.6)}$ with the wavelength of 8.5 um, as well as difference $V_s$ (=$V_{9.6}$-$V_{8.5(9.6)}$) between them calculated by control unit 23.

Referring to Fig. 3, when light emitting unit 11 starts emitting light having a wavelength of 9.6 um (Fig. 3 "emission on"), output $V_{9.6}$ with a wavelength of 9.6 um sharply rises. The output changes thereafter also, during the emission. Though output $V_{8.5}$ with a wavelength of 8.5 um does not show a sharp rise, the output changes during the emission. Since the light of 9.6 um wavelength is cut by the 8.5 um filter, only the thermal change of measured body 30 not containing the signal of emission light is reflected in output $V_{8.5(9.6)}$. Here, as a value showing a relative relationship between output $V_{9.6}$ and output $V_{8.5(9.6)}$, if output difference $V_s$ between them is employed for example, as shown in Fig. 3, output difference $V_s$ between them shows a substantially constant value during the emission. This is because the output change attributed to heat included in output $V_{9.6}$ has been corrected by the change in output $V_{8.5}$. It can be regarded that output difference $V_s$ (=$V_{9.6}$-$V_{8.5(9.6)}$) between output $V_{9.6}$ and output $V_{8.5(9.6)}$ representing the relative relationship between output $V_{9.6}$ and output $V_{8.5(9.6)}$ is the value being corrected to eliminate from output $V_{9.6}$ the effect of heat obtained from output $V_{8.5(9.6)}$.

Fig. 4 shows output $V_{9.6}$ from light receiving unit 17 with a wavelength of 9.6 um around each glucose concentration of 0g/dl, 0.5g/dl, and 1g/dl, and output difference $V_s$ (=$V_{9.6}$-$V_{8.5(9.6)}$) between output $V_{9.6}$ with a wavelength of 9.6 um and output $V_{8.5(9.6)}$ with a wavelength of 8.5 um.

As shown in Fig. 4, output $V_{9.6}$ varies and it is difficult to recognize the relationship between concentration and output. On the other hand, output difference $V_s$ (i.e., output $V_{9.6}$ corrected to eliminate the effect of heat obtained from output $V_{8.5(9.6)}$) shows small variation, and therefore the proportional relationship between glucose concentration and reflected light output can be obtained at control unit 23.

In the specific example presented above, output difference $V_s$ (=$V_{9.6}$-$V_{8.5(9.6)}$) is employed as a value corrected to eliminate from output $V_{9.6}$ the effect of heat obtained from output $V_{8.5(9.6)}$. However, correction of output $V_{9.6}$ is not limited to those performed with the above correction formula. For example, as another correction value, output ratio $V_s$=$V_{9.6}$/$V_{8.5(9.6)}$ is also effective. Still another correction value may be, when emission light is intense or when the 9.6 um filter and 8.5 um filter are different in the filter characteristics (such as half-width), output $V_{8.5(9.6)}$ provided with coefficient A, i.e., $V_s$=$V_{9.6}$-A•$V_{8.5(9.6)}$. In the following description, the correction value in which the effect of heat obtained from output $V_{8.5}$ is eliminated from output $V_{9.6}$ is expressed as $V_s$ = f($V_{9.6}$,$V_{8.5(9.6)}$), as a general formula.

Additionally, in the following description, it is described that the living body component measuring sensor emits light of a first wavelength specific to a measuring target living body component, and receives radiation from measured body 30. However, depending on the thickness of measured body 30, transmitted light may similarly be received for use in correction.

[First Embodiment]

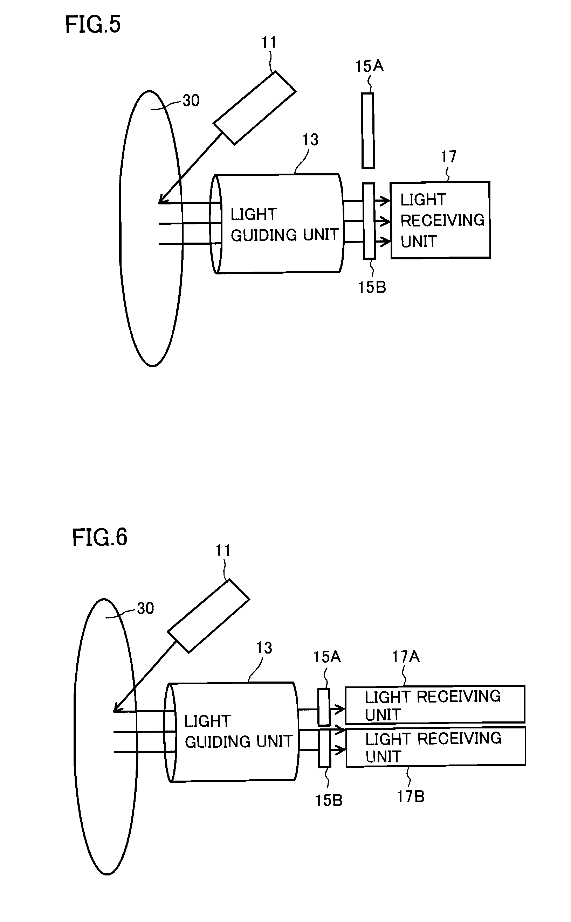

Referring to Fig. 5, living body component measuring sensor 10 includes a first filter 15A and a second filter 15B as filter 15.

First filter 15A is a filter transmitting light of an absorption wavelength (the aforementioned first wavelength) specific to a measuring target living body component, and second filter 15B is a filter transmitting light of a wavelength (the aforementioned second wavelength) of light not absorbed by the measuring target living body component. When the measuring target living body component is glucose, a 9.6 um filter and an 8.5 um filter correspond to first filter 15A and second filter 15B, respectively.

One of first filter 15A and second filter 15B is arranged at a position shielding light that has been guided from measured body 30 by light guiding unit 13 to light receiving unit 17 (see Fig. 5). After a very short period, their positions are switched so that the other filter is arranged at that position. Since it is preferable that light is received through both the filters for a plurality of times per one light emission, this switching is preferably performed for a plurality of times. While a mechanism for switching the positions of first filter 15A and second filter 15B is not limited to a specific mechanism in the present invention, one example may be a mechanism in which first filter 15A and second filter 15B are arranged on a disc, of which rotation alternately places first filter 15A and second filter 15B at the aforementioned position. Another specific example may be a mechanism in which first filter 15A and second filter 15B are provided on a plate, of which movement in a direction (in Fig. 5, up and down direction) perpendicular to the light guiding direction from measured body 30 alternately places first filter 15A and second filter 15B at the aforementioned position. With such a mechanism, control unit 23 outputs a control signal to a driving unit for switching the arrangement of first filter 15A and second filter 15B to control the switching.

Light emitting unit 11 emits the light of aforementioned first wavelength onto measured body 30. Light receiving unit 17 receives light of the first wavelength from measured body 30 through first filter 15A, and receives light of the second wavelength from measured body 30 through second filter 15B. Here, the light received at light receiving unit 17 through first filter 15A is scattering reflected light subjected to absorption by the measuring target living body component, and the light received through second filter 15B is blackbody radiation attributed to heat of measured body 30.

When the measuring target living body component is glucose, control unit 23 obtains output $V_{9.6}$ from light receiving unit 17 that is in accordance with a light amount of scattering reflected light of a wavelength of 9.6 um received through the 9.6 um filter when light of a wavelength of 9.6 um is emitted from light emitting unit 11, and output $V_{8.5(9.6)}$ from light receiving unit 17 that is in accordance with a light amount of blackbody radiation of a wavelength of 8.5 um received through the 8.5 um filter when light of a wavelength of 9.6 um is emitted from light emitting unit 11. Control unit 23 uses these outputs and corrects, as described above, output $V_{9.6}$, thereby obtaining the glucose concentration from which the effect of heat of measured body 30 is eliminated.

As a variation of the first embodiment, the configuration of living body component measuring sensor 10 may be the one shown in Fig. 6. That is, referring to Fig. 6, as a variation, living body component measuring sensor 10 may include a first light receiving unit 17A corresponding to first filter 15A and a second light receiving unit 17B corresponding to second filter 15B. Control unit 23 obtains output $V_{9.6}$ and output $V_{8.5(9.6)}$ by simultaneously receiving light at light receiving units 17A and 17B and performs the above correction, thereby obtaining the glucose concentration.

It is noted that, since the wavelength of the scattering reflected light is the same as that of the light emitted from light emitting unit 11, first filter 15A in light receiving unit 17 for receiving scattering reflected light of the light emitted from light emitting unit 11 may be an opening, instead of a filter. The same holds true for the other following embodiments.

[Second Embodiment]

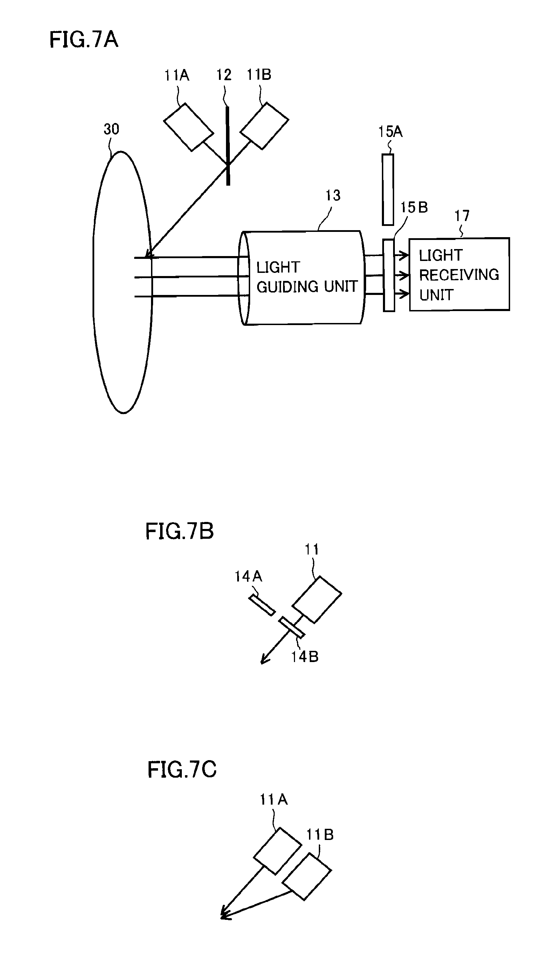

Referring to Fig. 7A, living body component measuring sensor 10 according to a second embodiment includes a first light emitting unit 11 A and a second light emitting unit 11 B as light emitting unit 11, and first filter 15A and second filter 15B as filter 15.

First light emitting unit 11A emits light of an absorption wavelength (aforementioned first wavelength) specific to a measuring target living body component, and second light emitting unit 11B emits light of a wavelength (the aforementioned second wavelength) of light not absorbed by the measuring target living body component.

First filter 15A is a filter transmitting the light of the absorption wavelength (the aforementioned first wavelength) specific to the measuring target living body component, and second filter 15B is a filter transmitting the light of the wavelength (the aforementioned second wavelength) of light not absorbed by the measuring target living body component.

When the measuring target living body component is glucose, the first wavelength and the second wavelength correspond to 9.6 um and 8.5 um, respectively, and a 9.6 um filter and an 8.5 um filter correspond to first filter 15A and second filter 15B, respectively.

Emission of the light of the first wavelength from first light emitting unit 11A and emission of the light of the second wavelength from second light emitting unit 11B are switched by mirror 12, so that measured body 30 is irradiated with one of them. Driving of mirror 12 is controlled by control unit 23. Similarly to living body component measuring sensor 10 according to the first embodiment, one of first filter 15A and second filter 15B is arranged at a position shielding light that has been guided from measured body 30 by light guiding unit 13 to light receiving unit 17 (see Fig. 7A). After a very short period, their positions are switched so that the other filter is arranged at that position. A specific mechanism therefor may be the same as the mechanism according to the first embodiment.

When the measuring target living body component is glucose, control unit 23 obtains output $V_{9.6}$ from light receiving unit 17 that is in accordance with a light amount of scattering reflected light of a wavelength of 9.6 um received through the 9.6 um filter as well as output $V_{8.5(9.6)}$ from light receiving unit 17 that is in accordance with a light amount of blackbody radiation of a wavelength of 8.5 um received through the 8.5 um filter when light of a wavelength of 9.6 um is emitted from first light emitting unit 11A, and output $V_{8.5}$ from light receiving unit 17 that is in accordance with a light amount of blackbody radiation of a wavelength of 8.5 um received through the 8.5 um filter as well as output $V_{9.6(8.5)}$ from light receiving unit 17 that is in accordance with a light amount of blackbody radiation of a wavelength of 9.6 um received through the 9.6 um filter when light of a wavelength of 8.5 um is emitted from second light emitting unit 11B. Control unit 23 uses these outputs and corrects output $V_{9.6}$, as described above. Here, with output $V_{8.5}$ attributed to light emission of an unabsorbed wavelength, the glucose concentration can be obtained from which not only the effect of the heat of measured body 30 but also the variation in the scattering state that is not recognized as heat is eliminated.

A specific example of the correction of output $V_{9.6}$ in the second embodiment may be as follows:

$$a \times \{( V_{9.6} - b \times V_{8.5(9.6)}) / (V_{8.5} - c \times V_{9.6(8.5)})\}$$

where a, b and c are coefficients.

It is noted that the configuration for switching the wavelength of light emitted from light emitting unit 11 onto measured body 30 is not limited to the configuration shown in Fig. 7A, and can be any other configuration. For example, as shown in Fig. 7B, as a configuration emitting light through filter 14A transmitting the first wavelength or through filter 14B transmitting the second wavelength, light emitting unit 11 may switch between filters 14A and 14B so that the wavelength of light emitted from light emitting unit 11 is switched between the first and second wavelengths. Alternatively, first light emitting unit 11A and second light emitting unit 11B may be arranged at the positions to both emit light onto measured body 30 as shown in Fig. 7C and emission from first light emitting unit 11A and emission from second light emission unit 11B are electrically switched so that the wavelength of light being emitted is switched.

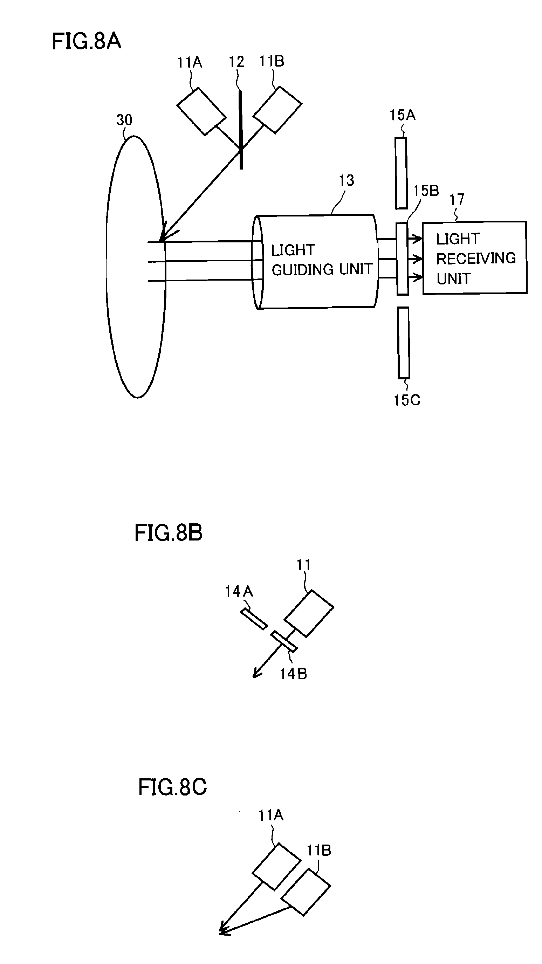

Referring to Fig. 8A, furthermore, living body component measuring sensor 10 according to a variation includes first filter 15A, second filter 15B, and a third filter 15C, as filter 15. First filter 15A is a filter transmitting light of an absorption wavelength (the aforementioned first wavelength) specific to a measuring target living body component. Second filter 15B is a filter transmitting light of a wavelength (the aforementioned second wavelength) of light not absorbed by the measuring target living body component. Third filter 15C is a filter transmitting light of an absorption wavelength (a third wavelength) of light not absorbed by the measuring target living body component but is specific to another component (an inhibiting component).

When the measuring target living body component is glucose, 9.6 um corresponds to the first wavelength, 8.5 um corresponds to the second wavelength, and 7.1 um corresponds to the third wavelength if the inhibiting component is albumin. A 9.6 um filter corresponds to first filter 15A, an 8.5 um filter corresponds to second filter 15B, and 7.1 um filter corresponds to third filter 15C.

When the light of the first wavelength is emitted from first light emitting unit 11A onto measured body 30, the light from measured body 30 is received via first filter 15A or second filter 15B by light receiving unit 17. When the light of the third wavelength is emitted from second light emitting unit 11B onto measured body 30, the light from measured body 30 is received via second filter 15B or third filter 15C by light receiving unit 17. Similarly to living body component measuring sensor 10 according to the first embodiment, one of first filter 15A and second filter 15B, or one of second filter 15B and third filter 15C is arranged at a position shielding light that has been guided by light guiding unit 13 from measured body 3 0 to light receiving unit 17 (see Fig. 8A). After a very short period, their positions are switched so that the other filter is arranged at that position. A specific mechanism therefor may be the same as the mechanism according to the first embodiment.

When the measuring target living body component is glucose, control unit 23 obtains output $V_{9.6}$ from light receiving unit 17 that is in accordance with a light amount of scattering reflected light of a wavelength of 9.6 um received through the 9.6 um filter as well as output $V_{8.5(9.6)}$ from light receiving unit 17 that is in accordance with a light amount of blackbody radiation of a wavelength of 8.5 um received through the 8.5 um filter when light of a wavelength of 9.6 um is emitted from first light emitting unit 11A, and output $V_{7.1}$ from light receiving unit 17 that is in accordance with a light amount of scattering reflected light of a wavelength of 7.1 um received through the 7.1 um filter as well as output $V_{8.5(7.1)}$ from light receiving unit 17 that is in accordance with a light amount of blackbody radiation of a wavelength of 8.5 um received through the 8.5 um filter when light of a wavelength of 7.1 um is emitted from second light emitting unit 11B. Control unit 23 uses these outputs and corrects output $V_{9.6}$, as described above. Thus, the glucose concentration can be obtained from which the effect of heat of measured body 30 and albumin variation are eliminated.

A specific example of the correction of output $V_{9.6}$ in the variation of the second embodiment may be as follows:

$$a \times \{( V_{9.6} - b \times V_{8.5(9.6)}) / (V_{7.1} - c \times V_{9.6(7.1)})\}$$

where a, b and c are coefficients.

It is noted that, in the variation also, the configuration for switching between the wavelengths of light emitted from light emitting unit 11 onto measured body 30 may be as shown in Figs. 8B and 8C.

[Third Embodiment]

The configuration of living body component measuring sensor 10 of a living body component measuring apparatus according to a third embodiment is similar to that of living body component measuring sensor 10 according to the second embodiment shown in Fig. 7A. In living body component measuring sensor 10 according to the present embodiment, control unit 23 provides control to rapidly switch between emission of light of the first wavelength from first light emitting unit 11 A and emission of light of the second wavelength from second light emitting unit 11B. Preferably the timing of switching between the first wavelength and the second wavelength is quicker than the thermal response of the living body being measured body 30.

When the measuring target living body component is glucose, light emitting unit 11 rapidly switches between light of a 9.6 um wavelength and light of an 8.5 um wavelength so that they are alternately emitted onto measured body 30. Control unit 23 obtains output $V_{9.6}$ from light receiving unit 17 that is in accordance with a light amount of scattering reflected light of a wavelength of 9.6 um received when light of a wavelength of 9.6 um is emitted from first light emitting unit 11A as well as output $V_{8.5}$ from light receiving unit 17 that is in accordance with a light amount of scattering reflected light of a wavelength of 8.5 um received when light of a wavelength of 8.5 um is emitted from second light emitting unit 11B, through first filter 15A being a 9.6 um filter.

Control unit 23 also obtains output $V_{8.5}$ from light receiving unit 17 that is in accordance with a light amount of scattering reflected light of a wavelength of 8.5 um received when light of a wavelength of 8.5 um is emitted from second light emitting unit 11B, and output $V_{8.5(9.6)}$ from light receiving unit 17 that is in accordance with a light amount of blackbody radiation of a wavelength of 8.5 um received when light of a wavelength of 9.6 um is emitted from first light emitting unit 11 A, through second filter 15B being a filter transmitting 8.5 um and not transmitting 9.6 um.

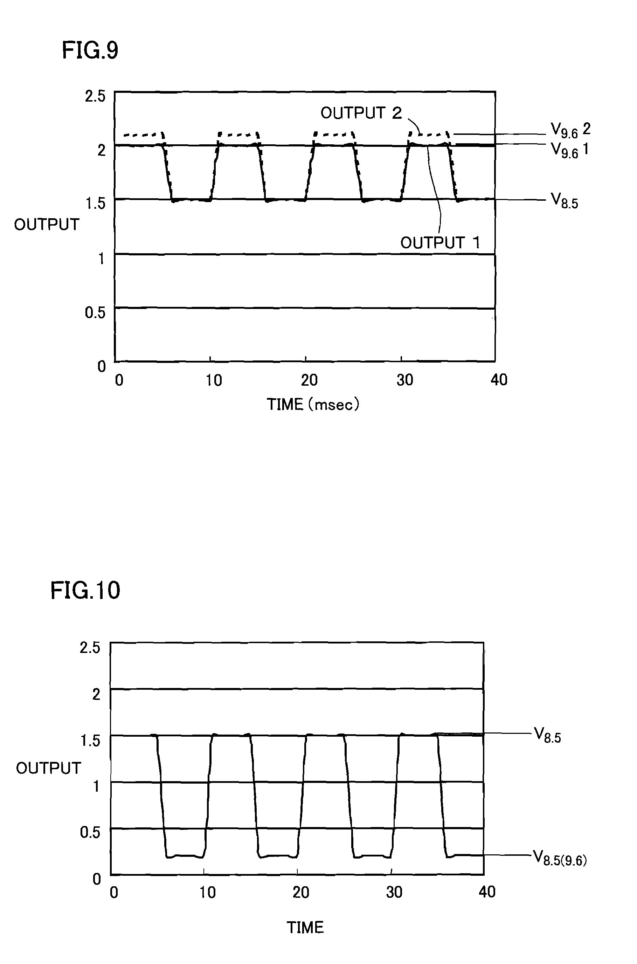

In Fig. 9, output 1 represents output $V_{9.6}$ and output $V_{8.5}$ with a certain glucose concentration. Output 2 represents output $V_{9.6}$ and output $V_{8.5}$ with a different glucose concentration. The horizontal axis of Fig. 9 indicates time lapse, showing that the wavelength of light emitted onto measured body 30 alternately switches between 9.6 um and 8.5 um at certain time intervals. As shown in Fig. 9, the wavelength of light emitted onto measured body 30 switching at the timing quicker than the thermal response of the living body that is measured body 30 provides the alternating waveform, in which output $V_{9.6}$ and output $V_{8.5}$ appear alternately. As shown by outputs 1 and 2, the change in the glucose concentration appears as the change in the amplitude of alternating components. The change in the amplitude of alternating components can precisely be detected by using a well-known small signal detection technique, for example by using a lock-in amplifier.

Fig. 10 shows output $V_{8.5(9.6)}$ when light of a wavelength of 9.6 um is emitted from first light emitting unit 11A onto measured body 30 and output $V_{8.5}$ when light of a wavelength of 8.5 um is emitted from second light emitting unit 11B onto measured body 30. As shown in Fig. 10, output $V_{8.5}$ represents the reflection state of measured body 30 relative to the emitted light of 8.5 um wavelength. Output $V_{8.5}$ contains, as compared with output $V_{8.5(9.6)}$, varying components such as scattering coefficient and moisture variation, which effect similar to the light of 9.6 wavelength except for absorption. Accordingly, by detecting output $V_{8.5}$, output $V_{8.5(9.6)}$ simultaneously with the difference between output $V_{9.6}$ and output $V_{8.5}$, and using them as correction terms in the correction formula presented above, the concentration of the measuring target living body component can be measured more precisely. That is, when obtaining the concentration of the measuring target living body component using the measurement value obtained with living body component measuring sensor 10 according to the third embodiment, the general formula for correction for obtaining correction value Vs from which the thermal effect is eliminated from output $V_{9.6}$ is expressed as Vs = f($V_{9.6}$ - $V_{9.6(8.5)}$, $V_{8.5}$, $V_{8.5(9.6)}$).

One example of the correction formula may be as follows, in which an output obtained through first filter 15A is $VS_1$ (=$V_{9.6}$ - $V_{8.5}$), an output obtained through second filter 15B is $VS_2$ (=$V_{8.5}$- $V_{8.5(9.6)}$), and coefficient is a:

$$a \times (VS_1/VS_2)$$

It should be understood that the embodiments disclosed herein are illustrative and non-restrictive in every respect. The scope of the present invention is defined by the terms of the claims, rather than the description and example above, and is intended to include any changes within the scope and meaning equivalent to the terms of the claims.

**Claims**

1. A living body component measuring apparatus, wherein a wavelength exhibiting specific absorption in a measuring target living body component is a first wavelength and a wavelength not exhibiting specific absorption in the living body component is a second wavelength, said living body component measuring apparatus comprising:

   a first light emitting unit (11, 11 A) emitting light of said first wavelength onto a measured body;
   a light receiving unit (15, 17) receiving light radiated from said measured body when said light of said first wavelength is emitted from said first light emitting unit, and outputting a signal according to a received light amount; and
   a concentration calculating unit (23) calculating concentration of said living body component based on said signal, wherein
   said light receiving unit includes a first light receiving unit (15A, 17, 17A) outputting a first signal according to a received light amount of said light of said first wavelength and a second light receiving unit (15B, 17, 17B) outputting a second signal according to a received light amount of said light of said second wavelength, and

said concentration calculating unit calculates the concentration of said living body component based on said first signal and said second signal.

2. The living body component measuring apparatus according to claim 1, wherein
said light of said first wavelength is mid-infrared light.

3. The living body component measuring apparatus according to claim 1, wherein
said concentration calculating unit calculates the concentration of said living body component based on said first signal and said second signal before and after said light of said first wavelength is emitted.

4. The living body component measuring apparatus according to claim 1, wherein
said second light receiving unit includes a filter (15B) transmitting said second wavelength, and receives said light of said second wavelength being transmitted through said filter from among light radiated from said measured body.

5. The living body component measuring apparatus according to claim 1, further comprising:

a second light emitting unit (11B) emitting said light of said second wavelength onto said measured body; and
switching means (12, 23) for switching between emission by said first light emitting unit and emission by said second light emitting unit; wherein
said light receiving unit further includes a third light receiving unit (15A, 17) outputting a third signal according to the received light amount of said light of said first wavelength radiated from said measured body and a fourth light receiving unit (15B, 17) outputting a fourth signal according to the received light amount of said light of said second wavelength radiated from said measured body, when said light of said second wavelength is emitted from said second light emitting unit, and
said concentration calculating unit calculates the concentration of said living body component based on said first to fourth signals.

6. The living body component measuring apparatus according to claim 1, wherein
a wavelength not exhibiting specific absorption in the living body component and being different from said second wavelength is a third wavelength, said living body component measuring apparatus further comprising:

a third light emitting unit (11B) emitting light of said third wavelength onto said measured body; and
switching means (12, 23) for switching between emission by said first light emitting unit and emission by said third light emitting unit; wherein
said light receiving unit further includes a fifth light receiving unit (15B, 17) outputting a fifth signal according to the received light amount of said light of said second wavelength radiated from said measured body and a sixth light receiving unit (15C, 17) outputting a sixth signal according to a received light amount of said light of said third wavelength radiated from said measured body, when said light of said third wavelength is emitted from said third light emitting unit, and
said concentration calculating unit calculates the concentration of said living body component based on said first signal, said second signal, said fifth signal, and said sixth signal.

7. The living body component measuring apparatus according to claim 1, further comprising:

a second light emitting unit (11B) emitting said light of said second wavelength onto said measured body; and
switching means (12, 23) for switching between emission by said first light emitting unit and emission by said second light emitting unit at a time interval that is shorter than thermal response of said measured body, wherein
said light receiving body further includes a fourth light receiving unit (15B, 17) outputting a fourth signal according to the received light amount of said light of said second wavelength radiated from said measured body when said light of said second wavelength is emitted from said second light emitting unit, and
said calculating unit calculates the concentration of said living body component based on a difference between said first signal and said fourth signal and a difference between said fourth signal and said second signal.

8. A living body component measuring sensor, wherein a wavelength exhibiting specific absorption in a measuring target living body component is a first wavelength and a wavelength not exhibiting specific absorption in the living body component is a second wavelength, said living body component measuring apparatus comprising:

a first light emitting unit (11, 11 A) emitting light of said first wavelength onto a measured body; and

a light receiving unit (15, 17) receiving light radiated from said measured body when said light of said first wavelength is emitted from said first light emitting unit, and outputting a signal according to a received light amount; wherein
said light receiving unit includes a first light receiving unit (15A, 17, 17A) outputting a first signal according to a received light amount of said light of said first wavelength and a second light receiving unit (15B, 17, 17B) outputting a second signal according to a received light amount of said light of said second wavelength.

9.  The living body component measuring sensor according to claim 8, further comprising:

a second light emitting unit (11B) emitting said light of said second wavelength onto said measured body; and switching means (12, 23) for switching between emission by said first light emitting unit and emission by said second light emitting unit; wherein
said light receiving unit further includes a third light receiving unit (15A, 17) outputting a third signal according to the received light amount of said light of said first wavelength radiated from said measured body and a fourth light receiving unit (15B, 17) outputting a fourth signal according to the received light amount of said light of said second wavelength radiated from said measured body, when said light of said second wavelength is emitted from said second light emitting unit.

10.  The living body component measuring sensor according to claim 8, wherein
a wavelength not exhibiting specific absorption in the living body component and being different from said second wavelength is a third wavelength, said living body component measuring apparatus further comprising:

a third light emitting unit (11B) emitting light of said third wavelength onto said measured body; and switching means (12, 23) for switching between emission by said first light emitting unit and emission by said third light emitting unit; wherein
said light receiving unit further includes a fifth light receiving unit (15B, 17) outputting a fifth signal according to the received light amount of said light of said second wavelength radiated from said measured body and a sixth light receiving unit (15C, 17) outputting a sixth signal according to a received light amount of said light of said third wavelength radiated from said measured body, when said light of said third wavelength is emitted from said third light emitting unit.

FIG.1

FIG.2

WAVELENGTH(um)

FIG.3

FIG.4

FIG.5

FIG.6

FIG.7A

11A    12    11B

30

15A

13

15B    17

LIGHT
GUIDING UNIT

LIGHT
RECEIVING
UNIT

FIG.7B

14A    11

14B

FIG.7C

11A    11B

FIG.8A

30

11A    12    11B

15A

13

15B    17

LIGHT
GUIDING UNIT

LIGHT
RECEIVING
UNIT

15C

FIG.8B

11

14A

14B

FIG.8C

11A

11B

## FIG.9

## FIG.10

## INTERNATIONAL SEARCH REPORT

| International application No. |
|---|
| PCT/JP2006/311530 |

A. CLASSIFICATION OF SUBJECT MATTER
*G01N21/35*(2006.01)i, *A61B5/1455*(2006.01)i

According to International Patent Classification (IPC) or to both national classification and IPC

B. FIELDS SEARCHED

Minimum documentation searched (classification system followed by classification symbols)
G01N21/00-G01N21/61, A61B5/145

Documentation searched other than minimum documentation to the extent that such documents are included in the fields searched
Jitsuyo Shinan Koho          1922-1996    Jitsuyo Shinan Toroku Koho    1996-2006
Kokai Jitsuyo Shinan Koho    1971-2006    Toroku Jitsuyo Shinan Koho    1994-2006

Electronic data base consulted during the international search (name of data base and, where practicable, search terms used)
JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | JP 2003-507703 A  (Regents of the University of Minnesota), 25 February, 2003 (25.02.03), Claims 1 to 8; Par. Nos. [0020] to [0033] & US 6486474 B1       & EP 1208366 A & WO 2001/013091 A2     & AU 6236800 A | 1-4,8 |
| X | JP 2000-74829 A  (Mitsui Chemicals, Inc.), 14 March, 2000 (14.03.00), Full text; Fig. 1 (Family: none) | 1,3,4,8 |
| X | JP 2005-102861 A  (Hitachi, Ltd.), 21 April, 2005 (21.04.05), Par. Nos. [0046] to [0049]; Fig. 10 & US 2005/070777 A1     & EP 1518493 A1 | 1,3,4,8 |

| ☒ | Further documents are listed in the continuation of Box C. | ☐ | See patent family annex. |
|---|---|---|---|

* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 August, 2006 (31.08.06) | 12 September, 2006 (12.09.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/311530

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 10-510180 A (Optiscan, Inc.), 06 October, 1998 (06.10.98), Full text; Fig. 1 & US 5615672 A   & EP 901339 A & WO 1996/017546 A1 | 1-10 |
| A | JP 2001-522625 A (Lightouch Medical, Inc.), 20 November, 2001 (20.11.01), Full text & US 6044285 A   & EP 1037553 A & WO 1999/023939 A1 | 1-10 |

Form PCT/ISA/210 (continuation of second sheet) (April 2005)

**EP 2 026 058 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10258036 A

- JP 5507866 A